# EUROPÄISCHE PATENTSCHRIFT

(11) **EP 1 270 573 B1**
(45) Veröffentlichungstag und Bekanntmachung des Hinweises auf die Patenterteilung: **01.09.2004**
(21) Anmeldenummer: 02013464.9
(22) Anmeldetag: 14.06.2002
(51) Int. Cl.: C07D 473/04, C07D 291/06, C07D 231/26, A23L 2/60, A61K 31/522, A61K 31/4152, A61P 25/28

(54) **Xanthin- und Phenazon-Acesulfam-H-Komplexe mit verbessertem Geschmack, Verfahren zu ihrer Herstellung und ihre Verwendung**
Xanthine and phenazone acesulfam-H complexes with improved taste, process for their preparation and their use
Complexes de xanthine et de phénazone avec l'acésulfame-H, à goût amélioré, leur procédé de préparation et leur application

(30) Priorität: 25.06.2001 DE 10130504
(43) Veröffentlichungstag der Anmeldung: 02.01.2003
(73) Patentinhaber: Nutrinova Nutrition Specialties & Food Ingredients GmbH, 65929 Frankfurt am Main (DE)
(72) Erfinder: Burgard, Andreas, Dr., 65929 Frankfurt am Main (DE)
(74) Vertreter: Schweitzer, Klaus, Dr.

(56) Entgegenhaltungen:
- EP-A- 0 956 780
- EP-A- 1 134 223
- DE-A- 2 000 210
- IT-B- 1 237 517

## Beschreibung

Die vorliegende Erfindung betrifft Xanthin- und Phenazon-Acesulfam-H-Komplexe bzw. Addukte mit verbessertem Geschmack, Verfahren zu deren Herstellung, ihre Verwendung sowie Arzneimittel enthaltend solche Verbindungen.

Xanthin-Derivate wie z.B. Propentofyllin und das Pentoxifyllin werden als Arzneimittelwirkstoffe in der Human- und Veterinärmedizin eingesetzt. Entsprechende Arzneimittel sind auf dem Markt.

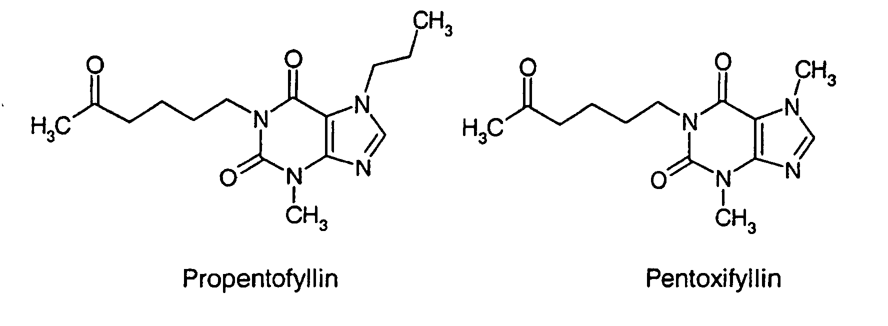

Propentofyllin wird seit mehreren Jahren in der geriatrischen Therapie beim Hund zur Verbesserung der Fließeigenschaften des Blutes im zerebralen und peripheren Bereich angewendet. In der Humanmedizin befindet sich Propentofyllin in der fortgeschrittenen klinischen Entwicklung für die Langzeitbehandlung von Patienten mit Alzheimer-Krankheit und vaskulärer Demenz. In klinischen Studien am Menschen verbesserte Propentofyllin die kognitiven Funktionen sowie die globalen Funktionen und Tätigkeiten des Alltagslebens bei Patienten mit Alzheimer-Krankheit und vaskulärer Demenz.

In DE-A 198 34 604 und WO-A 00/07541 wird außerdem die Verwendung von Propentofyllin zur Behandlung von Erektionsstörungen beschrieben.

Pentoxifyllin wird schon seit langer Zeit als Medikament unter verschiedenen Handelsnamen wie z.B. Trental®, Azupentat® zur Verbesserung der Hämodynamik bei Menschen eingesetzt, die an zerebralen, peripheren arteriellen und arteriovenösen Durchblutungsstörungen leiden.

Propentofyllin und Pentoxifyllin und andere Xanthine bzw. Xanthinderivate besitzen allerdings einen unangenehm bitteren Geschmack, so dass diese Verbindungen als Wirkstoffe in Medikamenten wegen des unangenehmen Geschmacks eine Aversion bei vielen Patienten hervorrufen können, insbesondere weil diese Medikamente sogar über Jahre eingenommen werden müssen.

1-Phenyl-2,3-dimethyl-3pyrazolin-5-on-Derivate aus der Substanzklasse der Phenazone wie z.B. das Phenazon (Dentigoa® N, Eu-Med mono®) selbst, das Propylphenazon (Arantil P®; R = -CH(CH₃)₂), Aminophenazon (Pyramidon®; R = - N(CH₃)₂) und das Metamizol (Novalgin®; R = -NCH₃-CH₂-SO₃Na) wirken analgetisch und antipyretisch. Die Phenazone sind bekannte Arzneimittelwirkstoffe und schmecken sehr bitter und eine Maskierung dieses unangenehmen Geschmacks wäre wünschenswert, insbesondere wenn Medikamente, die diese Wirkstoffe enthalten, mehrmals bzw. über einen längeren Zeitraum eingenommen werden müssen.

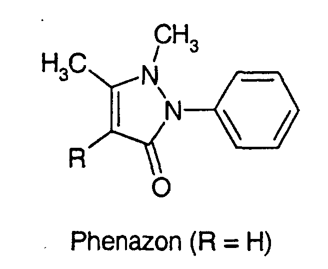

In DE-A 1 242 622, EP-A 0 046 506, WO-A 99/04822 und WO-A 00/12067 werden Verbindungen aus Süßstoff und Arzneimittelwirkstoffen mit verbessertem Geschmack beschrieben, wobei jeweils der Süßstoff und der Wirkstoff im molaren Verhältnis 1:1 vorliegen. Es handelt sich dabei um Säureadditionssalze bzw. um ionische Salzverbindungen, in denen das Süßstoffmolekül als Anion vorliegt. Diese Salze werden durch eine Säure-Base-Reaktion hergestellt, wobei der Süßstoff als Säure mit dem basisch reagierenden Wirkstoff umgesetzt wird.

Aufgabe der vorliegenden Erfindung war es, Xanthin-Derivate, wie z. B. Propentofyllin und Pentoxifyllin, und Phenazon-Derivate, wie z. B. Phenazon, in einer Form zur Verfügung zu stellen, die deren einfache Verwendung bei der Herstellung von z. B. Arzneimitteln oder anderen Zubereitungen ermöglicht, und bei der der bittere Geschmack dieser Wirkstoffe maskiert oder unterdrückt wird. Dadurch kann die Akzeptanz solcher Medikamente in der Human- und Veterinärmedizin deutlich erhöht und damit der Therapieerfolg, insbesondere bei häufiger oder Langzeiteinnahme, deutlich gesteigert werden.

Überraschenderweise wurde nun gefunden, dass Xanthinderivate wie z. B. Propentofyllin und Pentoxifyllin (nachfolgend Xanthin-Derivate genannt; Formel II) mit Acesulfam-H (Formel I), der zu Acesulfam-K korrespondierenden Säure, zu definierten Verbindungen reagieren können. Acesulfam (6-Methyl-3,4dihydro-1,2,3-oxathiazin-4-on-2,2-dioxid) ist insbesondere in der Form des Kaliumsalzes (Acesulfam-K) ein im Handel befindlicher Süßstoff. Sowohl Acesulfam-K als auch Acesulfam-H, die zu Acesulfam-K korrespondierende Säue, können nach bekannten Verfahren hergestellt werden (vgl. DE-A 2453 063, Angew. Chemie 85, 965-973 (1973), EP-A 0155 634). Bei der o.g. Umsetzung gelingt nicht nur allein die Herstellung von Komplexen allgemein aus einem Xanthin-Derivat und Acesulfam, sondern es lassen sich ebenso definierte Verbindungen aus einem Molekül Xanthin-Derivat und einem oder zwei Molekülen Acesulfam herstellen. Die angegebenen molaren 1:1- und 1:2-Verhältnisse von Xanthin-Derivat zu Acesulfam innerhalb dieser definierten Komplexe konnte mittels ¹H-NMR bestätigt werden. Überaschenderweise wurde per Röntgenstrukturanalyse festgestellt, dass es sich bei diesen Komplexen aus Xanthin-Derivat und Acesulfam nicht um Salze handelt, was eigentlich aus einer Säure-Base-Reaktion zu erwarten wäre, sondern um nichtionische Xanthin-Derivat-Acesulfam-Addukte, die bevorzugt im molaren Verhältnis 1:1 und 1:2 vorliegen. Diese Xanthin-Derivat-Acesulfam-Addukte liegen somit nicht als Salze vor, sondern sind charakterisiert durch Wasserstoff-Brückenbindungen zwischen einem Xanthin-Derivat-molekül und z. B. einem bzw. zwei Acesulfam-H-Molekülen. Das Acesulfam-H liegt nach Röntgenstrukturanalyse in diesen Addukten in seiner Enolform vor. Das nachfolgende Reaktionsschema dient zur Veranschaulichung dieses Sachverhalts:

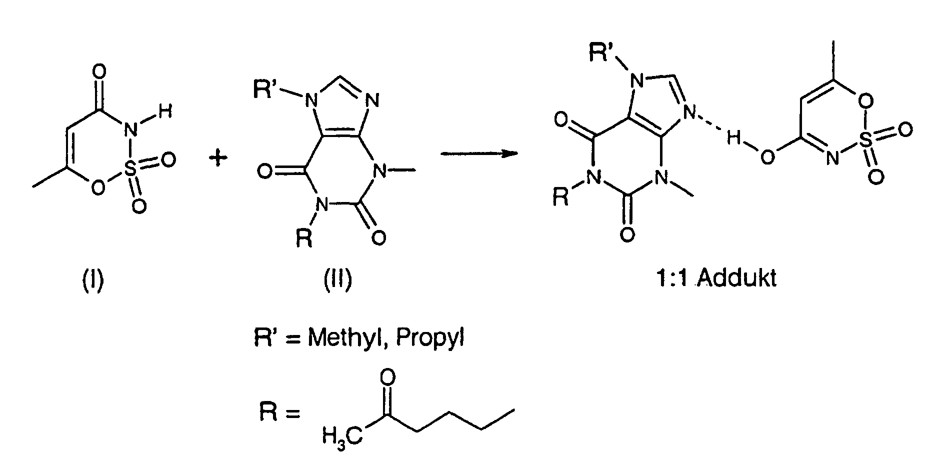

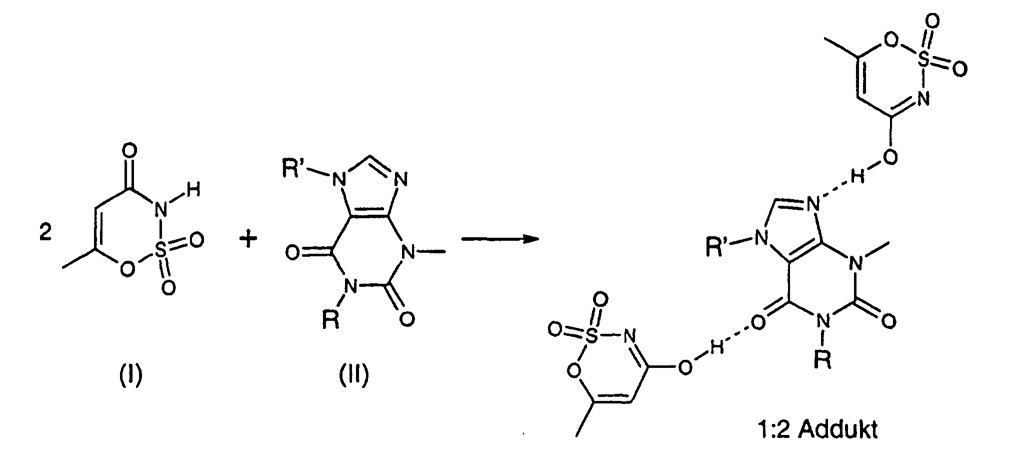

Überraschenderweise zeichnen sich alle diese Verbindungen sowie deren Solvate durch einen angenehmen Süßgeschmack aus, wobei die unangenehme Geschmackskomponente des Xanthin-Derivates beim 1:1-Addukt im Vergleich zum Xanthin-Derivat ohne Acesulfam deutlich gemindert ist und beim 1:2-Xanthin-Derivat-Acesulfam-Addukt zunächst durch einen zitrusartigen Süßgeschmack vollständig maskiert wird. Der sich anschließende leicht bittere Nachgeschmack des Xanthin-Derivates ist sehr deutlich vermindert.

Die Adduktbildung aus Xanthin-Derivat und Acesulfam bevorzugt in einem definierten Verhältnis führt nicht nur zu einer Verminderung bzw. Maskierung des unangenehmen bitteren Geschmacks der Xanthin-Derivate, sondern die definierten 1:1- bzw. 1:2-Xanthin-Derivat-Acesulfam-Addukte können nun sehr einfach z.B. in Kaugummis, Kautabletten oder Arzneimittel ohne die Gefahr einer Entmischung der Ausgangskomponenten eingearbeitet werden. Das Problem der Entmischung bei Transport oder bei dem Dosiervorgang entfällt ebenfalls.

Die Darstellung der Xanthin-Derivat-Acesulfam-Addukte gelingt sehr einfach z. B. aus Lösungen, bevorzugt aus wässrigen Lösungen des Xanthin-Derivates und des Acesulfam-H, in denen diese Stoffe bevorzugt im molaren Verhältnis 1:1 bzw. 1:2 vorliegen. Die erhaltenen Reaktionslösungen werden in geeigneter Weise, z. B. im Vakuum, vom Lösungsmittel befreit. Es resultieren jeweils die entsprechenden Xanthin-Derivat-Acesulfam-Addukte als farblose Kristalle, die nach ¹H-NMR-Spektroskopie im molaren Verhältnis 1:1- bzw. 1:2-Xanthin-Derivat-Acesulfam vorliegen. Auf diese Weise können auch die entsprechenden Solvate erhalten werden.

Als Lösungsmittel oder Lösungsmittelgemisch werden bevorzugt Wasser und/oder mit Wasser mischbare Lösungsmittel wie z. B. Alkohole verwendet. Die Reaktionstemperaturen liegen bevorzugt bei 20 bis 100 °C.

Überraschenderweise wurde weiterhin gefunden, dass Phenazon (Formel III) und Phenazon-Derivate mit Acesulfam-H, zu definierten Verbindungen reagieren können. Dabei gelingt z. B. auch die Herstellung eines Komplexes aus einem Molekül Phenazon und einem Molekül Acesulfam im molaren Verhältnis 1:1, was mittels ¹H-NMR bestätigt werden konnte. Diese definierten Komplexe sind besonders bevorzugt. Ferner umfasst die vorliegende Erfindung auch die entsprechenden Solvate sämtlicher Komplexverbindungen. Überraschenderweise wurde per Röntgenstrukturanalyse festgestellt, dass es sich bei diesen Komplexen aus Phenazon bzw. Phenazonderivaten und Acesulfam nicht um Salze handelt, was eigentlich aus einer Säure-Base-Reaktion zu erwarten wäre, sondern um ein nichtionisches Phenazon-Acesulfam-Addukt, das bevorzugt im molaren Verhältnis von 1:1 vorliegt. Diese Phenazon-Acesulfam-Addukte liegen somit nicht als Salze vor, sondern bilden über eine Wasserstoff-Brückenbindung zwischen einem Phenazon-molekül und einem Acesulfam-H-Molekül bevorzugt ein definiertes 1:1-Addukt. Das Acesulfam-H liegt nach Röntgenstrukturanalyse in diesen Addukten in seiner Enolform vor.

Das nachfolgende Reaktionsschema dient zur Veranschaulichung dieses Sachverhalts:

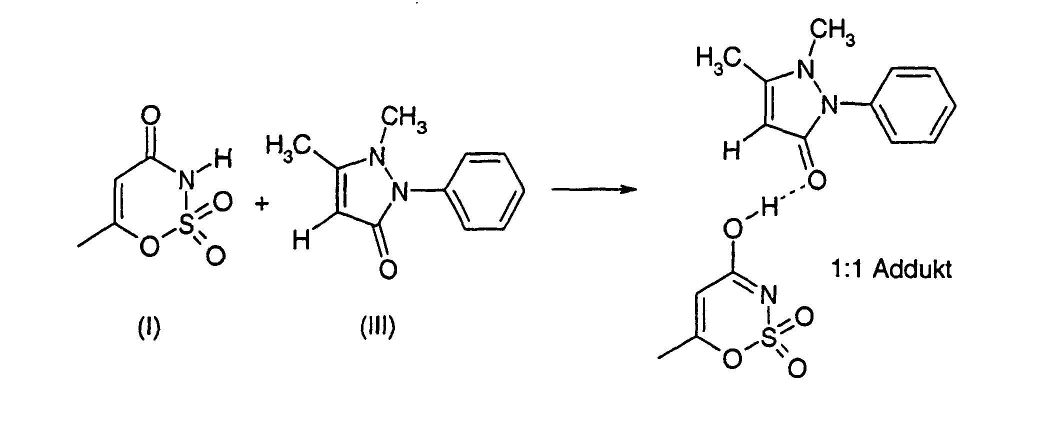

Überraschenderweise zeichnen sich die Phenazon-Acesulfam-Addukte sowie deren Solvate durch einen angenehmen Süßgeschmack aus, wobei die unangenehme Geschmackskomponente des Phenazons bzw. seiner Derivate beim 1:1-Addukt im Vergleich zum Phenazon bzw. Derivat ohne Acesulfam deutlich vermindert ist.

Die Adduktbildung aus Phenazonderivat und Acesulfam in einem definierten Verhältnis führt nicht nur zu einer Verminderung bzw. Maskierung des unangenehmen bitteren Geschmacks der Phenazonderivate, sondern die definierten 1:1-Addukte können nun sehr einfach z.B. in Kaugummis, Kautabletten oder Arzneimittel ohne die Gefahr einer Entmischung der Ausgangskomponenten eingearbeitet werden. Das Problem der Entmischung bei Transport oder bei dem Dosiervorgang entfällt ebenfalls.

Der Herstellung der Komplexe aus Phenazon und Süßstoff bzw. Acesulfam und deren Solvate erfolgt analog zur Herstellung der Xanthin-Derivat-Acesulfam-Addukte. Auf diese Weise können auch andere Phenazonderivate, wie z. B. Propyl- und Aminophenazon oder Metamizol, mit Acesulfam-H umgesetzt werden. Die entsprechenden Addukte und deren Solvate werden ebenfalls von der vorliegenden Erfindung umfasst.

Die Süßstoffsäure Acesulfam-H bildet somit überraschenderweise in ihrer Enolform mit heterocyclischen Verbindungen, die eine Carbonsäureamid-funktionalität besitzen, über eine Wasserstoffbrückenbindung definierte Komplexe. Diese Komplexbildung ist hier auch auf andere geeignete heterocyclische Systeme anwendbar, die noch nicht erwähnt sind, aber von der vorliegenden Erfindung ebenso mit umfasst sind wie die Verwendung solcher Komplexe zur Herstellung von Zubereitungen, z. B. in der Form von Arzneimitteln, und Arzneimittel bzw. Zubereitungen, welche solche Verbindungen enthalten.

Die bevorzugte Verwendung der definierten, in einem bestimmten molaren Verhältnis vorliegenden erfindungsgemäßen Komplexe bzw. Addukte stellt z. B. sicher, dass die daraus hergestellten Zubereitungen, wie z. B. Arzneimittel, die Xanthin- bzw. Phenazonderivate und den Süßstoff (Acesulfam) in der gewünschten einheitlichen bzw. festgelegten Menge enthalten. Werden diese Komponenten getrennt, z. B. als Premix, eingesetzt bzw. zudosiert, so kann es zu Entmischungen aufgrund unterschiedlicher Stoffeigenschaften kommen. D. h. eine Zubereitung kann z. B. zuviel und eine andere zu wenig Süßstoff enthalten.

Die Verarbeitung der erfindungsgemäßen Addukte in der Arzneimittelindustrie oder der Nahrungs- und Genussmittelindustrie erfordert gegenüber dem Einsatz von Xanthin- bzw. Phenazonderivat allein keine besonderen Vorkehrungen, sondern erfolgt nach den dort üblichen Verfahren. Dies gilt auch für Arzneimittelformulierungen bzw. für deren Herstellung.

Die vorliegende Erfindung umfasst somit auch Zubereitungen, bevorzugt Arzneimittel, z. B. in Form von Tabletten oder Tropfen, welche die erfindungsgemäßen Komplexe bzw. Addukte und/oder deren Solvate enthalten. Ferner werden auch entsprechende Vormischungen oder Premix, welche diese Verbindungen enthalten und bei der Herstellung der genannten Zubereitungen verwendet werden, von der Erfindung erfasst.

Die nachfolgenden Beispiele sollen die Erfindung näher erläutern.

### Beispiel 1

### Herstellung eines 1:1-Adduktes aus Pentoxifyllin und Acesulfam-H

In 20 ml Wasser werden 4 mmol (1,113 g) Pentoxifyllin mit 4 mmol (0,653 g) Acesulfam-H bei 55 °C gelöst. Das Reaktionsgemisch wird anschließend im Vakuum eingeengt. Es resultieren mit 100 % Ausbeute farblose Kristalle, welche laut ¹H-NMR als 1:1-Addukt vorliegen.
60-MHz-¹H-NMR (DMSO): δ(ppm) = 1.6 (m, 4H, CH₂-Pentoxifyllin), 2.2 (s, 3H, CH₃-Acesulfam), 2.35 (s, 3H, CH₃-CO-Pentoxifyllin), 2.6 (m, 2H, CO-CH₂-Pentoxifyllin), 3.55 (s, 3H, CH₃-Pentoxifyllin), 3,95 (m, 2H, CH₂-N-Pentoxifyllin), (4.05 (s, 3H, CH₃-Pentoxifyllin), 6.3 (s, 1H, CH-Acesulfam), 8.25 (s, 1H, CH-Pentoxifyllin), 11.8 (s, 1H, Acesulfam-H)

### Beispiel 2

### Herstellung eines 1:2-Adduktes aus Pentoxifyllin und Acesulfam-H

In 20 ml Wasser werden 4 mmol (1,113 g) Pentoxifyllin mit 8 mmol (1,306 g) Acesulfam-H bei 55 °C gelöst. Das Reaktionsgemisch wird anschließend im Vakuum eingeengt. Es resultieren mit 100 % Ausbeute farblose Kristalle, welche laut ¹H-NMR als 1 :2-Addukt vorliegen.
60-MHz-¹H-NMR (DMSO): δ(ppm) = 1.6 (m, 4H, CH₂-Pentoxifyllin), 2.2 (s, 6H, CH₃-Acesulfam), 2.35 (s, 3H, CH₃-CO-Pentoxifyllin), 2.6 (m, 2H, CO-CH₂-Pentoxifyllin), 3.55 (s, 3H, CH₃-Pentoxifyllin), 3,95 (m, 2H, CH₂-N-Pentoxifyllin), (4.05 (s, 3H, CH₃-Pentoxifyllin), 6.3 (s, 2H, CH-Acesulfam), 8.25 (s, 1H, CH-Pentoxifyllin), 11.8 (s, 2H, Acesulfam-H)

### Beispiel 3

### Herstellung eines 1:1-Adduktes aus Phenazon und Acesulfam-H

In 25 ml Methanol werden 35 mmol (6,72 g) Phenazon mit 35 mmol (5,71 g) Acesulfam-H bei 20° C gelöst. Das Reaktionsgemisch wird anschließend im Vakuum eingeengt. Es resultieren mit 100 % Ausbeute farblose Kristalle, welche laut ¹H-NMR als 1:1-Addukt vorliegen.
60-MHz-¹H-NMR (CDCl₃): δ(ppm) = 2.1 (s, 3H, CH₃-Acesulfam), 2.31 (s, 3H, CH₃-Phenazon), 3.29 (s, 3H, CH₃-N-Phenazon), 5.6 (s, 1H, CH-Acesulfam), 5.7 (s, 1H, CH-Phenazon), 8.4 (m, 5H, CH-Aromat), 13.05 (s, 1H, Acesulfam-H)

## Patentansprüche

1. Verbindung aus einer heterozyklischen Verbindung mit Carbonsäureamidfunktionalität und Acesulfam-H und deren Solvate

2. Verbindung nach Anspruch 1, **dadurch gekennzeichnet, dass** die heterozyklische Verbindung ein Xanthinderivat oder Phenazon oder ein Phenazonderivat ist.

3. Verbindung nach Anspruch 1 oder 2, **dadurch gekennzeichnet, dass** es sich um eine Verbindung aus Propentofyllin, Pentoxifyllin, Phenazon, Propylphenazon, Amino-phenazon oder Metamizol und Acesulfam-H handelt.

4. Verbindung nach einem oder mehreren der Ansprüche 1 bis 3, **dadurch gekennzeichnet,dass** das molare Verhältnis von Xanthinderivat, Phenazon oder Phenazonderivat zu Acesulfam-H 1 : 1 oder 1 : 2 beträgt.

5. Verbindung nach einem oder mehreren der Ansprüche 1 bis 4, **dadurch gekennzeichnet, dass** es sich um eine nicht-ionische Verbindung handelt.

6. Zubereitung enthaltend eine Verbindung nach einem oder mehreren der Ansprüche 1 bis 5.

7. Zubereitung nach Anspruch 6, **dadurch gekennzeichnet, dass** es sich um ein Human- oder Veterinärarzneimittel handelt.

8. Verwendung einer Verbindung nach einem oder mehreren der Ansprüche 1 bis 5 zur Herstellung einer Zubereitung nach Anspruch 6 oder 7.

9. Verfahren zur Herstellung einer Verbindung nach einem oder mehreren der Ansprüche 1 bis 5 durch Umsetzung der Ausgangskomponenten in dem gewünschten molaren Verhältnis in einem Lösungsmittel oder Lösungsmittelgemisch und gegebenenfalls anschließender Isolierung des gebildeten Reaktionsproduktes.

10. Verfahren nach Anspruch 9, **dadurch gekennzeichnet, dass** als Lösungsmittel Wasser und/oder mit Wasser mischbare Lösungsmittel verwendet werden.

## Claims

1. A compound of a heterocyclic compound having carboxamide functionality and acesulfame-H and its solvates.

2. A compound as claimed in claim 1, wherein the heterocyclic compound is a xanthine derivative or phenazone or a phenazone derivative.

3. A compound as claimed in claim 1 or 2, wherein it is a compound of propentofylline, pentoxifylline, phenazone, propylphenazone, aminophenazone or metamizole, and acesulfame-H.

4. A compound as claimed in one or more of claims 1 to 3, wherein the molar ratio of xanthine derivative, henazone or phenazone derivative to acesulfame-H is 1:1 or 1:2.

5. A compound as claimed in one or more of claims 1 to 4, wherein it is a nonionic compound.

6. A preparation comprising a compound as claimed in one or more of claims 1 to 5.

7. The preparation as claimed in claim 6, wherein it is a human or veterinary pharmaceutical.

8. The use of a compound as claimed in one or more of claims 1 to 5 for producing a preparation as claimed in claim 6 or 7.

9. A process for preparing a compound as claimed in one or more of claims 1 to 5 by reacting the starting components in the desired molar ratio in a solvent or solvent mixture and if appropriate subsequently isolating the reaction product formed.

10. The process as claimed in claim 9, wherein the solvent is water and/or water-miscible solvent.

## Revendications

1. Composé comprenant un composé hétérocyclique comportant une fonctionnalité d'amide d'acide carboxylique et acésulfam-H et ses composés de solvatation.

2. Composé selon la revendication 1, **caractérisé en ce que** le composé hétérocyclique est un dérivé de xanthine, phénazone ou un dérivé de phénazone.

3. Composé selon la revendication 1 ou 2, **caractérisé en ce qu'**il s'agit d'un composé de propentofyllin, pentoxifyllin, phénazone, propylphénazone, amino-phénazone ou metamizol et de acésulfam-H.

4. Composé selon l'une quelconque des revendications 1 à 3, **caractérisé en ce que** le ratio molaire entre dérivé de xanthine, phénazone ou dérivé de phénazone et acésulfam-H est de 1 : 1 ou de 1 : 2.

5. Composé selon l'une quelconque des revendications 1 à 4, **caractérisé en ce qu'**il s'agit d'un composé non ionique.

6. Composition comprenant un composé selon l'une quelconque des revendications 1 à 5.

7. Composition selon la revendication 6, **caractérisé en ce qu'**il s'agit d'une composition pharmaceutique à destination humaine ou vétérinaire.

8. Utilisation d'un composé selon l'une quelconque des revendications 1 à 5 pour la préparation d'une composition selon la revendication 6 ou 7.

9. Procédé de préparation d'un composé selon l'une quelconque des revendications 1 à 5, par réaction des produits de départ dans le ratio molaire souhaité dans un solvant ou un mélange de solvants et la cas échéant isolement subséquent du produit réactionnel formé.

10. Procédé selon la revendication 9, **caractérisé en ce que** l'on utilise à titre de solvant de l'eau et/ou des solvants miscibles avec l'eau.
